## Europäisches Patentamt
## European Patent Office
## Office européen des brevets

(19)

(11) Veröffentlichungsnummer: **0 034 301 B1**

(12) **EUROPÄISCHE PATENTSCHRIFT**

(45) Veröffentlichungstag der Patentschrift:
03.07.85

(51) Int. Cl.⁴: **G 01 N 33/536**

(21) Anmeldenummer: 81100775.6

(22) Anmeldetag: 04.02.81

(54) Verfahren zur Bestimmung von Immunkomplexen.

(30) Priorität: 14.02.80 DE 3005417

(43) Veröffentlichungstag der Anmeldung:
26.08.81 Patentblatt 81/34

(45) Bekanntmachung des Hinweises auf die Patenterteilung:
03.07.85 Patentblatt 85/27

(84) Benannte Vertragsstaaten:
AT BE DE FR IT

(56) Entgegenhaltungen:
EP - A - 0 005 978
DE - A - 2 736 805
DE - A - 2 749 956

IMMUNITÄT UND INFEKTION, Band 6, 1978 K. HELMKE et al. ″Nachweis zirkulierender Immunokomplexe mit der Laser-Nephelometrie″ Seiten 173 bis 179

Die Akte enthält technische Angaben, die nach dem Eingang der Anmeldung eingereicht wurden und die nicht in dieser Patentschrift enthalten sind.

(73) Patentinhaber: **BEHRINGWERKE AKTIENGESELLSCHAFT, Postfach 1140, D-3550 Marburg/Lahn (DE)**

(72) Erfinder: **Sieber, Axel, Dr., Sonnenhang 25, D-3550 Marburg/Lahn (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

**Beschreibung**

Die Erfindung betrifft ein Verfahren zum Nachweis von Immunkomplexen in Flüssigkeiten.

Es sind bereits Verfahren zum Nachweis und zur Bestimmung von Immunkomplexen (IK) in Blutserum bekannt. Diese Methoden haben den Nachteil, dass sie ungenau, unsicher, unspezifisch oder aufwendig sind. So beschreiben Helmke et al., Immunität und Infektion 6, 173 (1978), eine Methode, bei der die Trübung des Serums in einem Laser-Nephelometer gemessen, Polyäthylenglykol (PEG) oder ein spezifisches Antiserum zugegeben, zentrifugiert und die Trübung des Überstandes gemessen wird. Falls der Unterschied der beiden Trübungswerte genügend gross ist, soll daraus die Menge an Immunkomplexen bestimmt werden können. Es ist jedoch bekannt, dass Polyäthylenglykol auch andere Proteine neben den Immunkomplexen ausfällt und die Methode daher wenig spezifisch ist. Andererseits müssen Immunkomplex-haltige Seren nicht durch einen erhöhten Blindwert erkennbar sein, so dass eine einfache Differenzmessung nach Fällung mit PEG oder einem spezifischen Antiserum nicht in jedem Fall zum richtigen Ergebnis führt.

Ein Immunkomplex ist zusammengesetzt aus einer antigenen Komponente und einer Immunglobulin-Komponente, die ein Immunglobulin A, G und/oder M ist. In Immunkomplexen des Menschen besteht die Immunglobulin-Komponente naturgemäss aus humanem IgG, IgA und/oder IgM, gegen welche in bekannter Weise spezifische Antikörper in Tieren erzeugt und gewonnen werden können.

Es wurde nun überraschenderweise gefunden, dass die Geschwindigkeit, mit welcher ein solcher spezifischer Antikörper gegen eines der humanen Immunglobuline reagiert, verschieden ist, je nachdem, ob das humane Immunglobulin frei oder gebunden in einem Immunkomplex vorliegt. Der Antikörper reagiert meistens langsamer mit dem Immunglobulin, wenn es gebunden in einem Immunkomplex, als wenn es frei vorliegt.

Die Geschwindigkeit mit der ein Antikörper mit dem zugehörigen Antigen reagiert, kann in an sich bekannter Weise, beispielsweise aus der zeitlichen Zunahme der Lichtabsorption in einem Photometer oder auch der Trübung in einem Nephelometer bestimmt werden. Man kann sich dabei eines Schreibers bedienen, der das jeweilige Messsignal in Abhängigkeit von der Zeit mit einem Schreiber aufzeichnet oder auf einem Bildschirm sichtbar macht oder auch die Messdaten unmittelbar von einem Rechner verarbeiten lässt.

Das erfindungsgemässe Verfahren besteht darin, dass die Geschwindigkeit, mit der jeweils ein für eines der humanen Immunglobuline G, A oder M spezifischer Antikörper mit seinem Antigen in der Serumprobe reagiert, in Beziehung setzt zu der Geschwindigkeit, mit der derselbe Antikörper mit seinem Antigen in einem immunkomplexfreien Referenzserum reagiert, in welchem das entsprechende Antigen in derselben Konzentration wie in der zu untersuchenden Serumprobe vorliegt. Die Reaktionsgeschwindigkeit, mit der ein gegen einen der drei humanen Immunglobulintypen gerichteter Antikörper mit seinem korrespondierenden Immunglobulintyp reagiert, ist dann verändert, wenn dieser Immunglobulintyp gebunden in einem Immunkomplex vorliegt verglichen mit der Reaktionsgeschwindigkeit in einem Medium, in welchem dieser Immunoglobulintyp nicht in einem Immunkomplex vorliegt. Diese Veränderung der Reaktionsgeschwindigkeit wird in der Anfangsphase der Reaktion beobachtet, während sich die Geschwindigkeiten im Verlauf der Reaktion angleichen, unabhängig davon, ob das Immunglobulin in einem Immunkomplex gebunden oder frei vorliegt.

Aus dem bisher Gesagten geht bereits hervor, dass die Geschwindigkeit, mit der ein gegen einen der humanen Immunglobulintypen gerichteter Antikörper mit seinem Antigen reagiert, nur dann verändert ist, wenn sein Antigen ganz oder teilweise in einem Immunkomplex gebunden ist. Damit wird gleichzeitig angezeigt, welcher oder welche der drei Immunglobulintypen ganz oder zum Teil in einem Immunkomplex gebunden vorliegen.

Es wurden bei den Immunglobulinen G oder A immer verzögerte oder bei IgM teils verzögerte und teils schnellere Kinetiken beobachtet.

Weiterhin wurde gefunden, dass ebenfalls die Geschwindigkeit, mit welcher das im Immunkomplex vorliegende Antigen mit einem spezifisch gegen dieses gerichteten Antikörper reagiert, verringert ist. Dieser Befund kann in gleicher Weise zur Bestimmung der in einer Flüssigkeit vorliegenden Menge an Immunkomplexen dienen. Diese Ausgestaltung des erfindungsgemässen Verfahrens ist beispielsweise von Interesse bei Autoimmunerkrankungen, bei denen die Immunkomplexe körpereigene Proteine als Antigen enthalten.

Das Verfahren zum Nachweis von Immunkomplexen in einer Flüssigkeit ist dadurch gekennzeichnet, dass man die Geschwindigkeit bestimmt, mit der die in dieser Flüssigkeit vorliegenden Immunglobuline G, A und M oder das im Immunkomplex vorliegende Antigen jeweils mit einem hierfür spezifischen Antikörper reagieren, und mit derjenigen in Beziehung setzt, die gefunden wird, wenn der Antikörper mit dem Immunglobulin oder dem Antigen reagiert, wenn das Immunglobulin oder das Antigen in einer immunkomplexfreien Flüssigkeit vorliegen, wobei das Immunglobulin oder Antigen im Referenzserum in derselben Konzentration wie in der Probe vorliegt.

Die Geschwindigkeit wird zweckmässig aus dem Proportionalteil der Titrationskurve bestimmt.

Als immunkomplexfreie Flüssigkeit kann eine Standardlösung verwendet werden, beispielsweise das Serum eines gesunden Blutspenders oder eine Mischung von Seren gesunder Blutspender, die keine Immunkomplexe enthalten.

Im folgenden wird das Verfahren beispielhaft beschrieben:

In der zu untersuchenden Flüssigkeit werden die Konzentrationen von IgG, IgA und IgM mit Hilfe einer Methode zur quantitativen Proteinbestimmung, beispielsweise der radialen Immundiffusion (RID), Laser-Nephelometrie (LN) oder Turbidimetrie (TM), bestimmt. Bei den optischen Methoden LN und TM, wird die Konzentration aus dem Teil der Titrationskurve bestimmt, in welchem die Reaktionsgeschwindigkeit gegen 0 geht und die Kurve in der üblichen Darstellung in eine Horizontale übergeht. Ein solches Verfahren ist auch als Endpunktverfahren bekannt. Von einem normalen Referenzserum, in welchen keine Immunkomplexe vorhanden sind, von welchen jedoch die Konzentration an IgG, IgA und IgM bekannt sind, werden drei Verdünnungen so hergestellt, dass jeweils eine dieselbe Konzentration an IgG, IgA und IgM aufweist, wie sie in der zu untersuchenden Lösung gemessen wurden.

Man versetzt je eine Probe der auf Immunkomplexe zu untersuchenden Flüssigkeit in einer geeigneten Verdünnung jeweils mit einer bestimmten Menge eines für humanes Immunglobulin G, A oder M oder für das im Immunkomplex vorliegende Antigen spezifischen Antiserums und bestimmt die Geschwindigkeit der immunchemischen Reaktion. In gleicher Weise werden die entsprechenden Verdünnungen des Referenzserums, in denen die Immunglobuline oder das Antigen in derselben Konzentration wie in der zu untersuchenden Flüssigkeit vorliegen, behandelt.

Aus der Differenz der Reaktionsgeschwindigkeiten in der zu untersuchenden Flüssigkeit und dem Referenzserum wird die Menge an Immunkomplex bestimmt.

In einer bevorzugten Ausführungsform wird die Reaktionskinetik der Reaktion zwischen Antikörper und Immunglobulin oder Antigen aufgezeichnet. Der Endpunkt der Reaktion ist im allgemeinen nach 30 Minuten erreicht. Die Reaktion mit der entsprechenden Verdünnung des Referenzserums wird in gleicher Weise gemessen und die reaktionskinetische Kurve vorzugsweise über der entsprechenden Kurve der zu untersuchenden Flüssigkeit aufgezeichnet.

Falls die zu untersuchende Flüssigkeit keine Immunkomplexe enthält, verlaufen die Kurvenpaare von Untersuchungs- und Referenzflüssigkeit deckungsgleich. Liegen Immunkomplexe vor, so weicht mindestens eine der Kurven von der Kurve des Referenzserums ab. Die kinetische Kurve für die betreffende Reaktion in der zu untersuchenden Flüssigkeit weist dann meist eine geringere Steigung gegenüber der Referenzkurve auf. Ausnahmen können bei IgM-haltigen Immunkomplexen mit grösseren Steigungen auftreten.

Ähnliche Ergebnisse werden erhalten, wenn statt natürlicher Immunkomplexe künstliche Immunkomplexe, beispielsweise aus Tetanus-Toxoid und den entsprechenden Human-Antikörpern, die einem immunkomplexfreien Serum zugemischt wurden, der aggregiertes IgG, das bekanntlich grosse Ähnlichkeiten hinsichtlich seiner physikalisch-chemischen sowie immunchemischen Eigenschaften mit Immunkomplexen aufweist, verwendet werden.

Die Erfindung wird durch die folgenden Beispiele erläutert:

Beispiel 1
Messung von IK in einem Patientenserum

In einem Patientenserum wurden mit Hilfe der Laser-Nephelometrie (LN) 1090 mg/dl IgG, 825 mg/dl IgA und 137 mg/dl IgM gemessen. Das Patientenserum wurde mit physiologischer NaCl-Lösung 1 : 100 verdünnt und jeweils 100 µl mit jeweils 200 µl eines 1 : 5 verdünnten spezifischen Antiserums gegen Human-IgG, IgA bzw. IgM versetzt und die Reaktionskinetik der durch die Vergrösserung der Immunkomplexe entstehenden Trübung im Laser-Nephelometer über 30 min gemessen.

Ein IK-freies Referenzserum enthielt 1154 mg/dl IgG, 233 mg/dl IgA und 187 mg/dl IgM. Es wurde für die kinetische Messung von IgG 1 : 107, für IgA 1 : 28 und für IgM 1 : 136 verdünnt, so dass im entsprechenden Ansatz die gleichen Konzentrationen der drei Proteine vorlagen wie beim Patientenserum. Es wurden ebenfalls 100 µl der Serumverdünnung mit 200 µl der Antiserum-Verdünnung zur Reaktion gebracht und die Kinetik mit dem Laser-Nephelometer gemessen. Die Kurve der kinetischen Messung zeigte bei IgA einen deutlich flacheren Anstieg in dem Gebiet der grössten Reaktionsgeschwindigkeit verglichen mit dem Referenzserum. Die beiden übrigen Proteine zeigten einen identischen Verlauf bei Probe und Referenzserum. Klinisches Bild und Vergleichstests mit anderen Methoden bestätigen das Vorliegen von IK.

Beispiel 2
Messung von künstlichen Immunkomplexen

Zunächst wurde durch Mischen einer Verdünnungsreihe von Tetanus-Antikörpern humanen Ursprungs mit Tetanus-Toxoid im Laser-Nephelometer eine Heidelberger-Kurve gemessen, um das Gebiet des Antigen-Überschusses auszutitrieren, in dem lösliche IK vorliegen. Unter den gegebenen Bedingungen war das bei einer Mischung von 180 IE/ml Toxoid mit 50 IE/ml Antikörpern der Fall.

Die entsprechenden Mengenverhältnisse wurden in ein IK-freies Humanserum gegeben, das nun künstliche Human-Immunkomplexe enthielt. Das gleiche negative Humanserum wurde als Referenzserum verwendet, indem in eine weitere Probe die gleiche Menge nur an Tetanus-Antikörpern zugesetzt wurde.

So war sichergestellt, dass IK-Serum und Referenzprobe die gleichen Konzentrationen an IgG, IgA und IgM enthielten.

Der weitere Versuchsgang entspricht in seiner Durchführung Beispiel 1.

Die Messung ergab das Vorliegen von IK bei IgG und IgA während IgM-IK nicht nachweisbar waren.

Beispiel 3
Modellreaktion mit aggregiertem IgG

Ein IK-freies Human-Serum wurde 15 min auf 63 °C erwärmt, um das enthaltene IgG einer Hitzeaggregation zu unterwerfen. Anschliessend wurde das Serum 1 : 100 verdünnt und wie in Beispiel 1 beschrieben weiter untersucht. Als Referenzserum diente in diesem Falle das gleiche Serum ohne Hitzebehandlung. Es zeigte sich, dass das aggregierte IgG, analog wie bei IK, eine verzögerte Kinetik bei der Reaktion mit einem spezifischen Antiserum aufweist.

**Patentspruch**

1. Verfahren zum Nachweis von Immunkomplexen in einer Flüssigkeit, dadurch gekennzeichnet, dass man die Geschwindigkeit bestimmt, mit der die in dieser Flüssigkeit vorliegenden Immunglobuline G, A und M oder das im Immunkomplex vorliegende Antigen jeweils mit einem hierfür spezifischen Antikörper reagieren, und mit derjenigen in Beziehung setzt, die gefunden wird, wenn der Antikörper mit dem Immunglobulin oder dem Antigen reagiert, wenn das Immunglobulin oder das Antigen in einer immunkomplexfreien Flüssigkeit vorliegen, wobei das Immunglobulin oder Antigen im Referenzserum in derselben Konzentration wie in der Probe vorliegt.

**Claim**

A process for detecting immunocomplexes in a liquid, which comprises determining the rate at which the immunoglobulins G, A and M present in that liquid or at which the respective antigen present in such an immunocomplex react with an antibody specific for one of those immunoglobulins or that antigen, and relating that rate to that which is determined when that antibody reacts with that immunoglobulin or antigen present in an immunocomplex-free liquid, whereby the concentration of that immunoglobulin or antigen in the immunocomplex-free liquid is the same as in the sample.

**Revendication**

Procédé pour la détection de complexes immuns dans un fluide, caractérisé en ce qu'on détermine la vitesse avec laquelle les immunoglobulines G, A et M ou l'antigène présent dans le complexe immun réagissent chacun avec un anticorps spécifique, et qu'on la met en relation avec la vitesse observée lors de la réaction de l'anticorps avec l'immunoglobuline ou l'antigène lorsque l'immunoglobuline ou l'antigène est présent dans un liquide sans complexe immun, l'immunoglobuline ou l'antigène étant présent dans le sérum de référence à la même concentration que dans l'échantillon.